# EUROPEAN PATENT SPECIFICATION

(11) **EP 2 818 588 B1**
(45) Date of publication and mention of the grant of the patent: **01.05.2019**
(21) Application number: 14175082.8
(22) Date of filing: 30.06.2014
(51) Int. Cl.: D04H 3/00, D02G 3/04, D02G 3/38

(54) **Fabric and yarn with oxygenating properties**
Gewebe und Garn mit sauerstofffördernden Eigenschaften
Tissue et fil avec des propriétés oxygénantes

(30) Priority: 28.06.2013 IT MI20131095
(43) Date of publication of application: 31.12.2014
(73) Proprietor: Allergosystem S.r.l., 38068 Rovereto (TN) (IT)
(72) Inventor: Cipriani, Gianfranco, 38068 Rovereto TN (IT)
(74) Representative: Gislon, Gabriele

(56) References cited:
- WO-A1-03/083189
- US-A1- 2010 005 568
- US-A1- 2011 107 502
- US-B2- 6 725 691

## Description

The present invention relates to a fabric and a yarn having oxygenating properties. More in particular, the invention relates to an elastic yarn and a fabric incorporating said elastic yarn wherein fabric and elastic yarn comprise yarns obtained from fibers of polymeric resins having properties able to increase oxygenation of areas of the human body in contact or wrapped up with said fabric.

Synthetic polymeric based materials , able to increase the skin oxygenation level of a user when this comes in contact with such materials, are known in the art; in particular, said materials are used in the form of fibers which are then transformed in textile products worn by the user. For ease of description, such materials are identified below as "oxygenating polymers".

Thus, here and below by "oxygenating polymer" is meant a polymer able to raise the oxygen level in the blood of a person wearing a material, in particular a textile product, made-up at least partially of such an oxygenating polymer.

The above described effect is obtained by the addition of small percentages of materials of mineral basis, for example silicon oxide, aluminum oxide, titanium dioxide, etc., to the polymeric resin. Such a composition affects the electromagnetic radiations emitted by the body, in particular the infrared radiations, which are reflected toward the skin.

Furthermore, yarns obtained by fibers of such oxygenating polymers and fabrics obtained with such yarns, are known. For example, polymers used for a yarn of the above mentioned type are described in the Patent Application WO 2003/083189. In particular, the fibers used for oxygenating yarns are in general made of PET, polyethylene terephthalate, filled with inorganic particles that reflect and diffract the visible and the near-infrared light. For the sake of description simplicity, yarns obtained by this and other materials will be herein indicated as "yarns of oxygenating polymer" or "oxygenating yarns". A material having such characteristics is, for example, the polymeric yarn known as Celliant®, produced by Hologenix Company (USA).

Fabrics and clothing comprising yarns of oxygenating polymer, combined with yarns of cotton and/or other materials, are also known in the art to make fabrics and clothing. How the use of fabrics obtained by oxygenating yarns (i.e. fabrics comprising yarns of oxygenating polymers) can improve blood flow within the blood vessels under the skin and, consequently, the degree of skin oxygenation, has been verified in the art.

Oxygenation tests are in general carried out by detecting the TCPO₂ (transcutaneous oximetry) values and demonstrated an average increase of about 7% of levels measured when using an oxygenating fabric of Celliant type compared to an usual fabric. By the term Celliant fabric is meant a fabric containing at least 52% w/w Celliant yarn.

However, the increase of oxygenation level obtained by the garments made by the known oxygenating fabrics, is relatively limited and turned out to vary greatly depending on the applications; consequently, applications of oxygenating fabrics able to achieve the required technical effect are limited in number and type.

A further limitation in the application of fabrics in oxygenating polymers is when the fabric is used in the presence of skin lesions of the user.

Purpose of the present invention is to solve the problems discussed above and to provide a fabric and yarn able to raise the level of oxygenation increase and also able to be used on body parts having skin lesions, as for example those occurring in case of diabetes.

These and other purposes are achieved by the present invention, by a fabric according to claim 1. Preferred aspects are set forth in the relative dependent claims. In particular, the present invention concerns a fabric comprising an elastic yarn comprising an elastomeric core, i.e. a core made of an elastomer, covered at least partially by a non-elastomeric yarn, and in particular by a yarn of oxygenating polymer, to raise the oxygen level in the blood of a person wearing such a fabric. The oxygenating yarn has linear mass density comprised between 20,2 to 155,6 dtex (20 and 140 dens), whereas the elastomer forming the core of the yarn has linear mass density between 22,2 to 88,9 dtex (20 and 80 dens).

According to an aspect of the present invention, the yarn of oxygenating polymer is made as a continuous yarn.

According to a further aspect of the invention, the oxygenating yarn is a continuous yarn and is spiraled around the elastomer, with a number of windings per meter comprised between 400 and 800, preferably between 600 and 700.

Such a cover form turns out to be the most efficient. However it is not excluded the use of further methods to cover the elastomer by a yarn of oxygenating polymer, for example methods that are of minor quality, but that are simpler and more inexpensive. Suitable methods are those of spiraling that is carried out in a discontinuous yarn, e.g. through staple fibers, air jet spinning in a continuous yarn, and making a core-spun yarn, made of a discontinuous yarn.

Independently of the fiber and yarn type used in the covering, the linear mass density of the elastomer and oxygenating yarn are those mentioned above, i.e. the yarn of oxygenating polymer has linear mass density comprised between 22,2 to 155,6 dtex (20 and 140 dens), whereas the elastomer forming the central part of the yarn has linear mass density between 22,2 to 88,9 dtex (20 and 80 dens).

Thanks to such a solution, it is possible to reach a good degree of skin oxygenation of a user, in fact the portion of the front surface of the fabric occupied by an oxygenating polymer considerably increases, because the presence of the elastomer is masked by the covering obtained by means of the oxygenating polymer itself. Moreover, the presence of the elastomer covered having linear mass densities indicated above makes it possible to cover a greater skin area with oxygenating material, being the area covered by the fabric the same, compared to the known art. Furthermore, a continuous yarn material allows for a more efficient operation for covering the elastomer.

According to another aspect of the present invention, the fabric also comprises an antibacterial yarn. Such an antibacterial yarn assures a correct hygiene for the user, for example it allows for an antifungal effect in case the fabric is used for making a sock. The antibacterial yarn can be uniformly distributed in the fabric, but is preferably located in the areas of greatest interest and is placed so as to substantially contact the skin when the garment is worn. Also in this case, the presence of an elastic yarn as described above eases the fabric effectiveness.

According to an aspect of the present invention, the antibacterial yarn comprises at least one chitin derivative, for example chitosan. In a preferred embodiment, such an antibacterial yarn is manufactured with a weight percent of chitin derivatives greater than 20%, preferably greater than 40%. Among the different types of antibacterial yarns usable with the present invention, suitable yarns are for example antibacterial yarns comprising a chitosan polymer, such as those commercialized with the name of Crabyon®, or yarns derived from natural or artificial or synthetic cellulose fibers impregnated with chitosan, for example the fiber commercialized as TENCEL®-C of the LENZING company. Other types of yarns with biocidal properties, such as for example yarns impregnated with biocidal or biostatic solutions in general, such as colloidal Ag and the like, are usable.

Preferably, the antibacterial yarn has a linear mass density comprised between 84,4 to 492,1 (12 and 70 Ne), more preferably between 118,1 to 492,1 dtex (12 and 50 Ne); a preferred range is comprised between 196,8 to 295,3 dtex (20 and 30 Ne). According to an aspect of the present invention, the final fabric has a weight comprised in the range from 90 g/sqm to 270 g/sqm, preferably from 110 g/sqm to 250 g/sqm. This fabric is preferably a knit fabric and preferably the weight also comprises the portion made with antibacterial, i.e. biocidal, yarn.

As mentioned, the fabric can be weft and warp or a knit fabric. The structure of the fabric is of known type in the art, for what relates to the knit, suitable structures are for example those known as double weft fabric.

In an embodiment wherein there is an antibacterial yarn, the latter is on both sides of the fabric. The yarn arranged on the side intended to contact the skin preferably has the form of semi-rings or "loops" which extend, between one stitch and the subsequent one of the fabric of oxygenating yarn, in a similar way to a terry cloth or French Terry.

The semi-rings are arranged in a sufficiently loose way to allow the fabric above, made of oxygenating yarn, to perform its action.

Therefore, further object of the present invention is a garment made by, or comprising, a fabric as described above (with or without an antibacterial yarn) and the use of a sock made by the fabric of the invention for preventing and/or treating the diabetes lesions.

Other objects of the invention are a yarn according to claim 12, a garment according to claim 11 or 17.

The invention is particularly advantageous because it allows enhancing considerably the increase in the obtainable values of oxygenation compared to traditional fabrics. Moreover, the combination of oxygenating yarn with the antibacterial yarn allows to obtain garments, for example socks, eye masks and underwear, having biostatic, or biocidal properties, allowing to use oxygenating fabrics for treatment or prevention of diabetes lesions, without the garments causing bacterial infections.

It has also surprisingly been verified that oxygenation values measured as TCPO₂ are still present, and in general improved and increased, also in the presence of an antibacterial yarn arranged between the oxygenating fabric and the skin, as verifiable with the example below.

According to an aspect, the yarn comprises fibers of oxygenating polymer and fibers of antibacterial yarn, both preferably obtained from staple. Such a yarn, since it comprises both oxygenating polymer and antibacterial yarn, consequently has at the same time oxygenating and biocidal properties, and in a preferred embodiment it has English cotton yarn number comprised between 20 and 40 Ne.

In an embodiment, the fibers of oxygenating polymer are present in said yarn in a range comprised between 50% and 80% w/w, whereas the fibers of antibacterial yarn are present in a range comprised between 20% and 50% w/w.

According to an aspect of the invention, the fibers of oxygenating polymer have linear mass density comprised between 1.2 and 2.5 dtex, preferably the fibers are in discontinuous yarn with length comprised between 2.5 and 5 cm.

The fibers of antibacterial yarn preferably have, in turn, linear mass density comprised between 1.2 and 2.5 dtex and are preferably discontinuous with a length comprised between 2.5 and 5 cm.

Therefore, an object of the invention is also a fabric comprising a yarn according to one of the embodiments set forth above.

In particular, according to the present invention, it is possible to make a fabric with a yarn comprising fibers of oxygenating polymer and fibers of antibacterial yarn, said commingled yarn being at least partially spiraled around an elastomer.

Therefore, a further object of the invention is a garment comprising the fabric above. Such a solution is evidently advantageous compared to the known art. In particular, "commingled" yarns made-up 20% of fibers of antibacterial yarn, for example fibers of Crabyon®, combined with 80% of inert support material, for example the viscose textile fiber Micro Modal® commercialized by Lenzing, are known.

The "active" portion of the yarns of the known art is therefore limited to 20% of the yarn, and has an antibacterial function only. Conversely, according to the present invention, a high percentage, also up to 100%, of the "commingled" yarn plays an active function, that turns out being both an oxygenating and antibacterial function.

With reference to the attached figures, some illustrative and non-limitative embodiments of the present invention are presented below, wherein:
- figure 1 is a magnified and schematic view of a covering of an elastomer made of a fabric according to the present invention;
- figure 2 is a magnified and schematic view of a covering of an elastomer made of a fabric according to another embodiment of the present invention;
- figure 3 is a magnified and schematic view of a covering of an elastomer made of a fabric according to an additional embodiment of the present invention;
- figure 4 is a plan view of a garment according to example 2 of the present invention;
- figure 5 is a partial, schematic, sectional and not scaled, view of the garment in figure 4.

As mentioned, a fabric according to the present invention comprises a yarn of an oxygenating polymer 1 and a yarn of an elastomer 2; the elastomer 2 being at least partially, preferably completely, covered by the yarn of oxygenating polymer 1. Preferably, the elastomer has linear mass density comprised between 20 and 80 dtex, and the oxygenating polymer preferably has linear mass density comprised between 20 and 140 dtex.

In such a way, the fabric surfaces have a high amount of yarn of oxygenating polymer 1 within the fabric, amount that can also substantially reach 100% of the fabric surface, at the same time the presence of the elastomer 2 guarantees good mechanical performance, in particular referring to elasticity and adherence thereof to the skin of the user. Such factors contribute to an optimal oxygenating effect of the fabric, and in particular of a garment made by such a fabric.

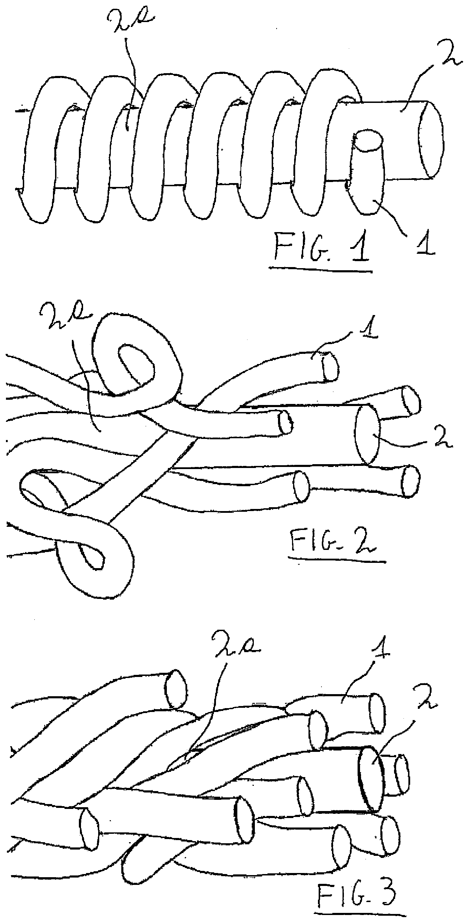

A first embodiment is shown with reference to figure 1.

In order to improve the covering process of the elastomer with the oxygenating polymer, and to make the finest possible knit fabric, in this embodiment an oxygenating polymer 1 made of continuous yarn, is used.

As it is known, for continuous yarn is meant a yarn made by several filaments of material having a length non-limited a priori, the filaments being cut with a length generally equal to the final yarn. This is differentiating from the discontinuous or "staple" yarn, consisting of a set of fibers having limited length, which are typically combined by twisting.

The continuous yarn allows for an optimal abrasion resistance of the fabric thanks to the use of microfibers and allows for a higher transpiration of the skin of a user. Finally, the continuous yarn turns out being lighter, weighing about one third of an equivalent discontinuous yarn.

In this embodiment, the yarn of oxygenating polymer 1 is spiraled around the elastomer 2, according to a method known as "spiraling".

Figure 1 just wants to be indicative of the result of the method, since it is meant that the areas 2a of elastomer 2, which are not covered by the oxygenating polymer 1 by spiraling, are in reality much more reduced compared to what has been shown, if not substantially absent, since the oxygenating polymer 1 is wound with very reduced pitch around the elastomer 2. In particular, the oxygenating polymer 1 is wound around the elastomer 2 with a number of windings per meter comprised in the range 400 - 800, preferably 600 - 700. Since the arrangement of the oxygenating polymer 1 around the elastomer 2 is regular and repetitive, it is possible to know a priori what percentage of elastomer 2 remains uncovered as a function of the number of windings.

Thus, the spiraling method turns out being the solution of higher quality compared to the two following embodiments illustrated with reference to figures 2 and 3, which conversely have the advantage of a higher simplicity and inexpensiveness.

In figure 2 it is shown a different possibility of covering the elastomer 2 by a yarn of an oxygenating polymer 1.

Some continuous yarns of oxygenating polymer 1 and an elastomer 2 are passed through an air jet arranging the yarns of oxygenating polymer 1 randomly around the core consisting of the elastomer 2. Such an operation turns out being quicker, simpler and more inexpensive of the spiraling but, due to the random arrangement of the oxygenating polymer 1, it guarantees a lower covering quality of the elastomer 2; consequently, the uncovered areas 2a of elastomer will have slightly higher size compared to the preceding embodiment.

In figure 3 it is shown an embodiment wherein a discontinuous yarn of oxygenating polymer 1 is used. To the detriment of advantages of the continuous yarn previously mentioned, the discontinuous yarn allows for making a core-spun yarn, wherein an elastomer 2 is irregularly covered by a set of discontinuous fibers of oxygenating polymer 1. Such a solution, not practicable through the continuous yarn, allows for a good minimization of the uncovered areas 2a of elastomer 2, and turns out being sufficiently inexpensive. However, the resulting yarn turns out having a linear mass density higher than one obtained by spiraling. Moreover, given the irregular arrangement of the oxygenating polymer, some areas 2a of the elastomer will remain uncovered following the processing.

In the spiraling processing, a discontinuous yarn can also be used, in an embodiment similar to that described regarding figure 1. Also in that case the number of windings per meter is preferably comprised between 400 and 800, and more preferably between 600 and 700. Preferably, the fabric of the present invention is a knit fabric. For creating the fabric according to the invention, in particular a knit fabric, it is possible to use elastic yarns only, i.e. yarns comprising a core of elastomer 2 covered by a yarn of oxygenating polymer 1, for example by one of the methods showed in the three previously described embodiments.

However in the fabric the use of yarns of oxygenating polymer that are non-elastic yarns, i.e. "free", together with the yarn formed by the elastomer covered by an oxygenating polymer, is possible. By the terminology "non-elastic yarn", or "free yarn" is meant a yarn of oxygenating polymer that is lacking a core made of the elastomer 2, i.e. they are substantially inelastic yarns. The amount of non-elastic yarns compared to the elastic yarns is selected as a function of the required elasticity for the final fabric, the ratio "number of non-elastic yarns/number of elastic yarns" is in general in the range from 1/1 up to 4/1, preferably from 1/1 to 2/1.

In the sock described below in example 1, for each filament of covered elastomer 2, one filament of "free" oxygenating polymer is used.

According to a further embodiment of the present invention, two or more "free" filaments of oxygenating polymer are coupled and twisted, and preferably two filaments are twisted by a method known as "doubling".

Thanks to this it is possible to increase at the same time the mechanical characteristics of the "free" yarn and its volume, thus increasing the amount of radiation it intercepts, with a surprisingly positive effect on oxygenating capabilities of the fabric.

The garments of examples 3 and 4 are made by this solution, and show the oxygenating effectiveness of the fabric.

The pairs of doubled filaments are preferably used as "free" filaments only, i.e. they do not act as a cover for the elastomer.

However it is not excluded the use of pairs of doubled filaments of oxygenating polymer to cover, preferably by spiraling, an elastomer.

Each filament of the pair has linear mass density comprised between 11,1 to 155,6 dtex (10 and 140 dens). However, to avoid that the pair of doubled filaments have excessive linear mass density, using filaments with linear mass density comprised between 11,1 to 100 dtex (10 and 90 dens) is preferable.

So far it has been discussed about "an" oxygenating polymer, but it is not intended to exclude the use of two or more types of oxygenating polymers. For example, a first oxygenating polymer may be used as a fabric backing, whereas a second oxygenating polymer may be used as a cover of the elastomer.

According to an aspect of the invention, it is possible to add antibacterial yarn to the fabric. As specified above, such a yarn is made of materials adapted to reduce the bacteria proliferation when the yarn is contacted with the skin. Such materials generally operate on the absorption degree of the skin moisture, in particular preventing the skin to dry. Furthermore, they prevent the formation of bad smells. Preferably, the antibacterial yarns comprise fibers of animal origin. The chitin derivative fibers proved to be particularly suitable for the invention, for example chitosan polymers, with which materials known as Crabyon® and Tencel®-C, previously cited or equivalent products, are obtained, among others.

As mentioned above, the chitin-derived fiber is preferably present at least at 20% w/w of the antibacterial yarn, and more preferably at least at 40% w/w of the antibacterial yarn.

In general, the antibacterial yarn can be uniformly distributed in the fabric, or alternatively its use can be concentrated in confined areas only.

Furthermore, it is possible to compose the fabric in such a way that the antibacterial yarn is present onto the fabric surface contacting the skin only, whereas the oxygenating polymer 1 is arranged on the opposite surface, so as to form a double fabric.

The antibacterial yarns can be, in their turn, partially or totally placed so as to cover an elastomer 2. In such a case, since the known antibacterial yarns are available in discontinuous yarn, they are processed by spiraling or to form a core-spun yarn around the elastomer 2, in a way similar to the two corresponding embodiments previously described pertaining the covering of the elastomer 2 through the oxygenating polymer 1.

It has been further verified how a fabric according to the present invention can be advantageously used even if arranged inside a garment. An example of such an application is provided in Example 2 described below.

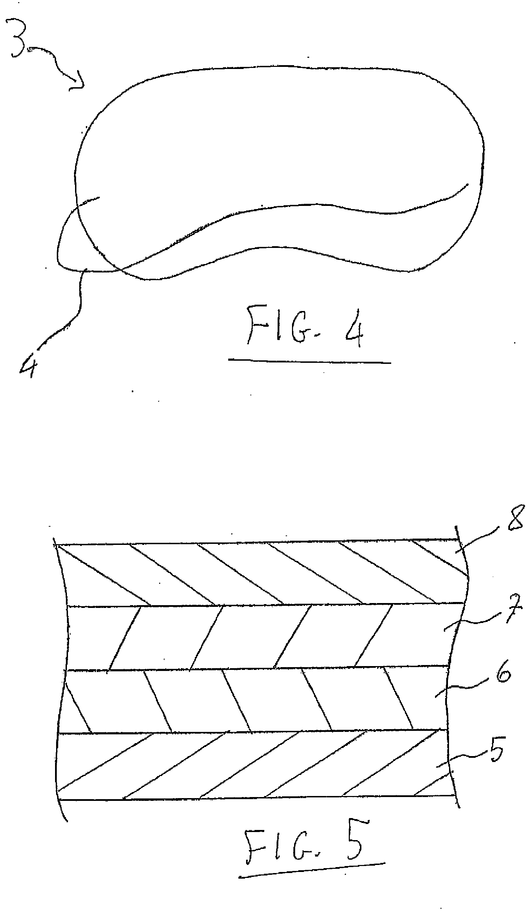

According to an embodiment of the present invention, shown in a schematic way in figures 4 and 5, a fabric 6 comprising an elastomer 2 covered at least partially by an oxygenating polymer 3 can be interposed between a fabric 5 comprising antibacterial yarn and a covering layer 8. Preferably the covering layer 8 has in turn oxygenating properties. Also with reference to Example 2, an internal layer 7 of wadding obtained from fibers of the oxygenating polymer 3 can be present.

According to the known art of manufacturing oxygenating yarns, the continuous multifilament yarns of oxygenating polymer, gathered in bundles consisting of a plurality of filaments, are subjected to interlacing during production, to create a number of cohesion points among the filaments composing the yarn, and provide improved mechanical characteristics, and particularly toughness. Such an operation, known in the art, consists for example of inserting the filaments composing the yarn into a pressurized air jet, shaking the filaments so as to cause bumps between each other in order to form connection points among the filaments.

In an embodiment of the invention, the yarn of oxygenating polymer is a continuous multifilament yarn not subjected to interlacing.

Such a solution involves the advantage of increasing the reflecting surface of the yarn compared to a traditional yarn, similarly to what happens with the previously described doubling method, whereby, other than in addition, it can be used as an alternative solution to filament doubling of oxygenating polymer.

The use of a non-interlaced oxygenating yarn has further the advantage to maintain a reduced weight, compared to a doubled yarn. Furthermore, the non-interlaced yarn is lacking the normal roughness instead present in the interlaced yarn, with advantages for a user comfort since such a yarn is intended to contact the skin of the user himself.

Therefore, a fabric comprising a continuous yarn of oxygenating polymer not subjected to interlacing turns out being innovative; such a non-interlaced yarn can be used as a covering yarn of the polymeric core and/or as free, non-elastic yarn.

The preferred ranges, in terms of presence of the above described constitutive elements within a fabric made according to the present invention, are reported below. The values are expressed by weight percent:
- oxygenating polymer: 50% - 99%. Preferably such a range is comprised between 90% and 99%. In case of high presence of antibacterial yarn, an optimal range in terms of presence of oxygenating polymer has been verified being between 60% and 80%.
- elastomer: 1% - 5%. Preferably such a range is comprised between 1% and 3%. In case of high presence of antibacterial yarn, an optimal range in terms of presence of elastomer has been verified being between 1% and 5%.
- antibacterial yarn: 0% - 40%. Preferably such a range is comprised between 0% and 35%. A preferred amount of antibacterial yarn is comprised between 20% and 30% w/w.

The fabric according to the present invention generally has a grammage comprised between 90 g/sqm and 270 g/sqm, preferably between 110 g/sqm and 250 g/sqm. According to an alternative embodiment of the present invention, fibers of oxygenating polymer and fibers of antibacterial yarn are processed together to form a unique filament essentially comprising oxygenating fibers and antibacterial fibers in the proportions indicated here below. In the case of fibers of antibacterial polymer obtained from chitosan, such fibers are mixed with fibers of oxygenating polymer, for example Celliant®, and processed to form the desired yarn.

In particular, according to a preferred embodiment, fibers of oxygenating polymer having linear mass density comprised between 1.2 and 2.5 dtex (preferably equal to about 1.8 dtex) and length comprised between 2.5 and 5.0 cm (preferably between 3.5 and 4.0 cm) are obtained from staple of oxygenating polymer, whereas fibers of similar linear mass density (1.2 - 2.5 dtex) and length (2.5 - 5.0 cm) are obtained from staple of antibacterial yarn.

Such fibers are processed to form a yarn, for convenience herein identified as "commingled" yarn, having English cotton yarn number comprised between 147, 6 to 295,3 dtex (20 and 40 Ne), in such commingled yarn the fibers of oxygenating polymer are present in a range comprised between 50% and 80%, and the fibers of antibacterial yarn are present in a percentage comprised between 20% and 80%, where the percentages are expressed by weight.

The so obtained commingled yarn is a further object of the present invention; it can be used as is or as covering yarn of an elastomer core, in a way similar to what previously described for the yarn of oxygenating polymer. Preferably such commingled yarn is spiraled around an elastomer.

With this type of commingled yarn it is possible to make very light fabrics, having at the same time high oxygenating and biocidal properties.

In a further embodiment of the present invention, a fabric, and thus a garment, comprises at least one backing comprising at least one elastomeric core, having linear mass density comprised between 22,2 to 88,9 dtex (20 and 80 dens), covered by oxygenating polymer having linear mass density comprised between 11,1 to 155,6 dtex (10 and 140 dens), preferably between 22,2 to 100 dtex (20 and 90 dens). Preferably, such a backing alternates elastomers covered by oxygenating polymer with "free" yarns of oxygenating polymer, made by means of pairs of doubled filaments of oxygenating polymer.

Each filament of the pair preferably has linear mass density comprised between 11,1 to 155,6 dtex (10 and 140 dens), more preferably between 22,2 to 100 dtex (20 and 90 dens).

According to a further aspect, the ratio between the number of covered elastomers and the pairs of doubled filaments of oxygenating polymer is preferably comprised between 1/1 and 1/4, more preferably between 1/1 and 1/2.

In a further embodiment of the invention, such a fabric or garment further has also a "commingled" yarn as previously described, i.e. yarn made by combining fibers of oxygenating polymer and fibers of antibacterial polymer. Both fibers are discontinuous and obtained from staple.

As mentioned, such a "commingled" yarn has English cotton yarn number comprised between 147,6 to 295,2 dtex (20 and 40 Ne), with fibers of oxygenating polymer present in a range comprised between 50% and 80%, and fibers of antibacterial yarn present in a percentage comprised between 20% and 50% (percentage expressed by weight). The sock of the following example 4 is made by the present embodiment. Such a commingled yarn is preferably added to the backing, in order to cover it both internally and externally, in a "sandwich" conformation. Preferably, such a commingled yarn is added to the backing only in some areas of the fabric or garment. For example, in case of a sock, such a commingled yarn can be added to the backing at the area of the sock intended to cover a user's foot sole.

In other words, such a commingled yarn is added to the backing preferably in limited areas, so that such areas have at least three layers in section, i.e. one central layer formed by the backing comprising an elastic yarn as described above, and two layers, an upper one and a lower one with respect to the backing, which have commingled oxygenating-antibacterial yarn.

### Example 1

A sock according to the invention is made by the following composition:
- oxygenating polymer: 70% w/w Celliant ®.
- elastomer spiraled with Celliant®: 2% w/w Lycra;
- Antibacterial yarn: 28% w/w Crabyon®.

In particular, the sock backing is made with a knit fabric having for each filament of Celliant 75/36/0 dtex ("free" or "non-elastic" yarn) a yarn of Celliant® 75/36/0 dtex spiraled onto Lycra 20/1 dens (elastic yarn).

In the areas of the toe and heel of the sock, two filaments of Crabyon 30/1 Ne are added to the backing composition of the above described knit.

The result of an experiment comparing the degree of oxygenation, measured as partial pressure of oxygen in the skin (TCPO₂), of the same individual wearing a sock identical to that of the invention but made with standard yarns (normal sock) and a sock according to the present example 1 (Oxygen sock), is reported below.

**Table 1**

| | | | | | | | | | | | |
|---|---|---|---|---|---|---|---|---|---|---|---|
| **Time** | 0 | 10 | 20 | 30 | 40 | 50 | 60 | 70 | 80 | 90 | ***min*** |
| **Normal sock** | 55 | 55 | 55 | 54 | 55 | 56 | 55 | 55 | 56 | 56 | ***mmHg*** |
| **Oxygen sock** | 57 | 58 | 58 | 57 | 59 | 58 | 62 | 61 | 61 | 62 | ***mmHg*** |
| **Δ** | 3.6 | 5.4 | 5.4 | 5.5 | 7.3 | 14.3 | 12.7 | 10.9 | 8.9 | 10.7 | **%** |

As can be seen from the table above, whereas with the use of a standard sock the partial pressure of oxygen of the foot skin remains substantially constant over time, with the use of a sock according to the present invention the partial pressure of oxygen of the foot skin shows a substantial increase during time.

The oxygenation increases obtained with the fabric according to the present invention reach 14% at regimen, i.e. after the garments have been worn for sufficient time; this effect is particularly surprising if considering that increases in oxygenation obtained by the known oxygenating fabrics are at most of about 7% compared to a "normal sock".

### Example 2

An eye mask 3, illustrated in figures 4 and 5, is made by overlapping four layers of textile material 5, 6, 7, 8, joined by sewing.

The layers of textile material 5, 6, 7 and 8 are schematically showed with the same thickness, for ease of visualization, even if the layers of textile material have different thickness between each other.

In particular, in the outer side the eye mask 3 has, on one side intended to contact the user's eyes, a first fabric 5 made by a yarn knit of TENCEL®-C having English cotton yarn number 30/1 Ne. On the contrary, the opposite side is made by a fabric 8 made of 100% polyester of linear mass density 70 dens.

Internally to the two external layers described above, two further internal layers 6 and 7 are present. In particular, in contact with TENCEL®-C a knit fabric 6 made according to the present invention, is present, and in particular a fabric 6 made by 50% yarn TENCEL®-C and 50% yarn Celliant®, obtained from staple and spiraled onto Lycra®. Both yarns have English cotton yarn number equal to 30/1 Ne.

On the contrary, the internal layer 7, in contact with the polyester, is composed of wadding in 100% Celliant.

The four layers 5, 6, 7, 8 in textile material have substantially the form of the final eye mask and, once overlapped, are joined by a sewing made at the edge of the fabrics themselves.

Typically an elastic 4 is added to the eye mask to allow it to be worn by the user.

### Example 3

A sock according to the invention is made by the following composition:
- oxygenating polymer: 98% w/w Celliant ®.
- elastomer spiraled with Celliant®: 2% w/w Lycra;

In particular, the sock is a tights having for each pair 75/72/2 dtex of Celliant doubled filaments, i.e. the yarn defined as "non-elastic" or "free", one filament of Lycra 40 Ne spiraled with one single filament of Celliant 75/72 dtex, i.e. the elastic yarn.

The result of two experiments comparing the degree of oxygenation, measured as partial pressure of oxygen in the skin (TCPO₂), of a pair of individuals wearing a sock identical to that of the invention but made with standard yarns (normal sock) and a sock according to the present example 3 (Oxygen sock), is reported below.

**Table 2.1**

| | | | | | | | | | | | |
|---|---|---|---|---|---|---|---|---|---|---|---|
| **Time** | 0 | 10 | 20 | 30 | 40 | 50 | 60 | 70 | 80 | 90 | ***min*** |
| **Normal sock** | 58 | 59 | 57 | 58 | 59 | 59 | 60 | 60 | 59 | 59 | ***mmHg*** |
| **Oxygen sock** | 58 | 60 | 63 | 65 | 66 | 67 | 65 | 62 | 63 | 65 | ***mmHg*** |
| **Δ** | 0.0 | 1.7 | 10.5 | 12.1 | 11.9 | 13.6 | 8.3 | 3.3 | 6.8 | 10.2 | **%** |

**Table 2.2**

| | | | | | | | | | | | |
|---|---|---|---|---|---|---|---|---|---|---|---|
| **Time** | 0 | 10 | 20 | 30 | 40 | 50 | 60 | 70 | 80 | 90 | ***min*** |
| **Normal sock** | 58 | 61 | 59 | 63 | 65 | 63 | 63 | 66 | 67 | 66 | ***mmHg*** |
| **Oxygen sock** | 63 | 68 | 68 | 69 | 71 | 72 | 73 | 70 | 72 | 74 | ***mmHg*** |
| **Δ** | 8.6 | 11.5 | 15.3 | 9.5 | 9.2 | 14.3 | 15.9 | 6.1 | 7.5 | 12.1 | **%** |

### Example 4

A sock according to the invention is made by the following composition:
- oxygenating polymer: 80% w/w Celliant®.
- elastomer spiraled with Celliant®: 2% w/w Lycra;
- Crabyon® fibers: 18% w/w;

The sock has a backing made by pairs 75/72/2 dtex of Celliant doubled filaments, i.e. the "free" yarn, which are alternate, with a 1:1 ratio, with filaments of Lycra 40 Ne spiraled with a single Celliant filament 75/72 dtex, i.e. the elastic yarn. At the sole and heel of the foot, a "commingled yarn" according to the present invention is added to the backing, internally and externally, and in particular a 30/1 Ne yarn made by 50% w/w Crabyion fibers and 50% Celliant fibers.

The result of two experiments comparing the degree of oxygenation, measured as partial pressure of oxygen in the skin (TCPO₂), of a pair of individuals wearing a sock identical to that of the invention but made with standard yarns (normal sock) and a sock according to the present example 4 (Oxygen sock), is reported below.

**Table 3.1**

| | | | | | | | | | | | |
|---|---|---|---|---|---|---|---|---|---|---|---|
| **Time** | 0 | 10 | 20 | 30 | 40 | 50 | 60 | 70 | 80 | 90 | ***min*** |
| **Normal sock** | 45 | 49 | 50 | 52 | 54 | 53 | 50 | 48 | 49 | 46 | ***mmHg*** |
| **Oxygen sock** | 47 | 48 | 53 | 54 | 56 | 57 | 58 | 57 | 59 | 59 | ***mmHg*** |
| **Δ** | 4.4 | -2.0 | 6.0 | 3.8 | 3.7 | 7.5 | 16.0 | 18.8 | 20.4 | 28.3 | **%** |

**Table 3.2**

| | | | | | | | | | | | |
|---|---|---|---|---|---|---|---|---|---|---|---|
| **Time** | 0 | 10 | 20 | 30 | 40 | 50 | 60 | 70 | 80 | 90 | ***min*** |
| **Normal sock** | 45 | 46 | 49 | 48 | 50 | 49 | 46 | 47 | 46 | 47 | ***mmHg*** |
| **Oxygen sock** | 48 | 49 | 51 | 53 | 55 | 54 | 56 | 55 | 54 | 55 | ***mmHg*** |
| **Δ** | 6.7 | 6.5 | 4.1 | 10.4 | 10.0 | 10.2 | 21.7 | 17.0 | 17.4 | 17.0 | **%** |

In particular the benefit of the addition of areas made by "commingled" yarn, allowing to reach thresholds of oxygenation increase 20% higher than a standard sock, with peaks of 28%, can be seen, in particular following an extended use.

The articles of clothing obtained with, or at least partially comprising, a fabric according to the invention, in addition to those of medical type, are for example undergarments, socks, T-shirts, caps and eye masks.

## Claims

1. A fabric comprising an elastic yarn having an elastomeric core and a non-elastomeric yarn, the latter being arranged around said elastomeric core to cover it at least partially, **characterized in that** said non-elastomeric yarn covering at least partially the elastomeric core is a yarn obtained from fibers of oxygenating polymer which is suitable to raise the oxygen level in the blood of a person wearing said fabric; said elastomeric core having a linear mass density comprised in the range from 22,2 to 80,9 dtex (20 to 80 dens) and said oxygenating yarn having a linear mass density comprised between 11.1 to 155.6 dtex (10 and 140 dens), more preferably between 22.2 to 100 dtex (20 and 90 dens).

2. A fabric according to claim 1, **characterized by** further comprising non-elastic yarns of an oxygenating polymer.

3. A fabric according to claim 1 or 2, wherein said oxygenating yarn is spiraled around said elastomeric core, preferably the number of windings of said oxygenating yarn around the elastomeric core being comprised in the range from 400 to 800 windings per meter.

4. A fabric according to any claim 1 to 3, wherein said oxygenating yarn is a continuous yarn.

5. A fabric according to any one of the preceding claims, wherein said elastic yarn and/or said non-elastic yarn comprise a doubled yarn made of filaments of oxygenating polymer.

6. A fabric according to any one of the preceding claims, further comprising an antibacterial yarn preferably having English cotton yarn number comprised between 84.4 to 492.1 dtex (12 and 70 Ne), preferably between 118.1 to 492.1 dtex (12 and 50 Ne), even more preferably between 196.8 to 295.3 dtex (20 and 30 Ne).

7. A fabric according to claim 6, wherein said antibacterial yarn comprises a fiber comprising at least one chitin derivative, and wherein said chitin derivative is present in said antibacterial yarn in a weight percent equal to or greater than 20%.

8. A fabric according to claim 6 or 7, wherein said yarn of said oxygenating polymer is present in an amount comprised between 50% and 99% w/w of the whole fabric, said core-spun elastomeric yarn is present in an amount comprised between 1% and 5% w/w and said antibacterial yarn is present in an amount comprised between 0% and 40% w/w.

9. A fabric according to one of the preceding claims, having a weight comprised in the range from 90 g/sqm to 270 g/sqm, preferably from 110 g/sqm to 250 g/sqm.

10. A fabric according to claim 1, **characterized in that** said non-elastomeric yarn is a continuous yarn of oxygenating polymer, wherein the filament fibers of said polymer are in a non-interlaced or non-constrained condition.

11. A garment made of a fabric or comprising it according to any one of claims 1 to 10.

12. An elastic yarn comprising an elastomeric core and a non-elastomeric yarn, said non-elastomeric yarn being arranged around said elastomeric core to cover it at least partially, **characterized in that** said non-elastomeric yarn is a yarn obtained from fibers of an oxygenating polymer, suitable to raise the oxygen level in the blood of a person wearing said yarn, said elastomeric core having a linear mass density comprised in the range from 22.2 to 80.9 dtex (20 to 80 dens) and said non-elastomeric yarn having a linear mass density comprised in the range from 11.1 to 155.6 dtex (10 and 140 dens).

13. A yarn according to claim 12, wherein said non-elastomeric yarn covering at least partially the elastomeric core is made as a continuous filament and is spiraled around said core, preferably with a number of windings per meter of said oxygenating polymer around the elastomeric core comprised in the range from 400 to 800 twists per meter.

14. A yarn according to claim 12, wherein the non-elastomeric yarn is a yarn comprising fibers of oxygenating polymer and fibers having antibacterial properties.

15. A yarn according to claim 14 wherein the amount of said fibers of oxygenating polymer in said yarn is comprised in a range from 50% to 80% w/w, and the amount of said fibers with antibacterial properties in said yarn is comprised in a range from 20% and 50% w/w.

16. A yarn according to any claim 12 to 15, which is a core-spun yarn wherein said fibers of oxygenating polymer are staple fibers.

17. A garment comprising a yarn according to any claim 12 to 16.

## Patentansprüche

1. Gewebe, umfassend ein elastisches Garn mit einem elastomeren Kern und einem nicht-elastomeren Garn, wobei das letztere um den elastomeren Kern herum angeordnet ist, um diesen zumindest teilweise zu bedecken, **dadurch gekennzeichnet, dass** das den elastomeren Kern zumindest teilweise bedeckende, nicht-elastomere Garn ein aus Fasern eines Sauerstoff-anreichernden Polymers erhaltenes Garn ist, welches geeignet ist, den Sauerstoffgehalt im Blut einer das Gewebe tragenden Person zu erhöhen; wobei der elastomere Kern eine lineare Massendichte aufweist, welche im Bereich von 22,2 bis 80,9 dtex (20 bis 80 den) umfasst es, und das Sauerstoff anreichernde Garn eine lineare Massendichte aufweist, welche zwischen 11,1 und 155,6 dtex (10 und 140 den) umfasst ist, vorzugsweise zwischen 22,2 und 100 dtex (20 und 90 den).

2. Gewebe gemäß Anspruch 1, **dadurch gekennzeichnet, dass** es ferner nicht-elastische Garne aus einem Sauerstoff-anreichernden Polymer umfasst.

3. Gewebe gemäß Anspruch 1 oder 2, wobei das Sauerstoff-anreichernde Garn spiralförmig um den Elastomerkern herumgewunden ist, wobei vorzugsweise die Anzahl der Windungen des Sauerstoff-anreichernden Garns um den Elastomerkern im Bereich von 400 bis 800 Windungen pro Meter umfasst ist.

4. Gewebe gemäß einem der Ansprüche 1 bis 3, wobei das Sauerstoff-anreichernde Garn ein kontinuierliches Garn ist.

5. Gewebe gemäß einem der vorhergehenden Ansprüche, wobei das elastische Garn und/oder das nicht-elastische Garn ein gedoppeltes Garn umfassen, welches aus Filamenten eines Sauerstoff-anreichernden Polymers hergestellt ist.

6. Gewebe gemäß einem der vorhergehenden Ansprüche, ferner umfassend ein antibakterielles Garn, vorzugsweise aufweisend eine englische Nummerierung für Baumwollgarne, welche zwischen 84,4 und 492,1 dtex (12 und 70 Ne), vorzugsweise zwischen 118,1 und 492,1 dtex (12 und 50 Ne), und noch bevorzugter zwischen 196,8 und 295,3 dtex (20 und 30 Ne) umfasst ist.

7. Gewebe gemäß Anspruch 6, wobei das antibakterielle Garn eine Faser umfasst, welche mindestens ein Chitin-Derivat umfasst, und wobei das Chitin-Derivat im antibakteriellen Garn bei 20% oder mehr Gewichtsprozent vorliegt.

8. Gewebe gemäß Anspruch 6 oder 7, wobei das Garn des Sauerstoff-anreichernden Polymers bezogen auf das gesamte Gewebe in einer Menge umfasst zwischen 50 und 99 Gew.-% vorliegt, wobei das elastomere Umspinnungsgarn in einer Menge umfasst zwischen 1 und 5 Gew.-% vorliegt und das antibakterielle Garn in einer Menge umfasst zwischen 0 und 40 Gew.- % vorliegt.

9. Gewebe gemäß einem der vorhergehenden Ansprüche, mit einem Gewicht umfasst im Bereich von 90 g/m² bis 270 g/m², vorzugsweise im Bereich von 110 g/m² bis 250 g/m².

10. Gewebe gemäß Anspruch 1, **dadurch gekennzeichnet, dass** das nicht-elastomere Garn ein kontinuierliches Garn eines Sauerstoff-anreichernden Polymers ist, wobei sich die Filamentfasern des Polymers in einem nichtverflochtenen oder nicht-gespannten Zustand befinden.

11. Kleidungsstück, hergestellt aus einem oder umfassend ein Gewebe gemäß einem der Ansprüche 1 bis 10.

12. Elastisches Garn, umfassend einen elastomeren Kern und ein nichtelastomeres Garn, wobei das nicht-elastomere Garn um den elastomeren Kern herum angeordnet ist, um diesen zumindest teilweise zu bedecken, **dadurch gekennzeichnet, dass** das nicht-elastomere Garn ein aus Fasern eines Sauerstoff-anreichernden Polymers erhaltenes Garn ist, dazu geeignet, den Sauerstoffgehalt im Blut einer das Garn tragenden Person zu erhöhen, wobei der elastomere Kern eine lineare Massendichte aufweist, welche im Bereich von 22,2 bis 80,9 dtex (20 bis 80 den) umfasst ist und das nicht-elastomere Garn eine lineare Massendichte aufweist, welche zwischen 11,1 und 155,6 dtex (10 und 140 den) umfasst ist.

13. Garn gemäß Anspruch 12, wobei das den Elastomerkern zumindest teilweise bedeckende, nicht-elastomere Garn als kontinuierliches Filament hergestellt und spiralförmig um den Kern herumgewunden ist, vorzugsweise mit einer Anzahl von Windungen pro Meter des Sauerstoff-anreichernden Polymers um den Elastomerkern umfasst im Bereich von 400 bis 800 Windungen pro Meter.

14. Garn gemäß Anspruch 12, wobei das nicht-elastomere Garn Fasern aus einem Sauerstoff-anreichernden Polymer und Fasern mit antibakteriellen Eigenschaften umfasst.

15. Garn gemäß Anspruch 14, wobei die Menge der Fasern des Sauerstoff-anreichernden Polymers im Garn in einem Bereich von 50 bis 80 Gew.-% umfasst ist und die Menge der Fasern mit antibakteriellen Eigenschaften im Garn im Bereich von 20 und 50 Gew.-% umfasst ist.

16. Garn gemäß einem der Ansprüche 12 bis 15, bei dem es sich um ein Umspinnungsgarn handelt, wobei die Fasern aus einem Sauerstoff-anreichernden Polymer Stapelfasern sind.

17. Kleidungsstück, umfassend ein Garn gemäß einem der Ansprüche 12 bis 16.

## Revendications

1. Tissu comprenant un fil élastique ayant une âme élastomère et un fil non-élastomère, ce dernier étant disposé autour de ladite âme élastomère pour la recouvrir au moins partiellement, **caractérisé en ce que** ledit fil non-élastomère recouvrant au moins partiellement l'âme élastomère est un fil obtenu à partir de fibres de polymère oxygénant qui est adapté pour augmenter le taux d'oxygène dans le sang d'une personne portant ledit tissu ; ladite âme élastomère ayant une densité massique linéaire comprise dans la plage de 22,2 à 80,9 dtex (20 à 80 dens) et ledit fil oxygénant ayant une densité massique linéaire comprise entre 11,1 et 155,6 dtex (10 et 140 dens), de manière plus préférée entre 22,2 et 100 dtex (20 et 90 dens).

2. Tissu selon la revendication 1, **caractérisé en ce qu'**il comprend en outre des fils non-élastiques d'un polymère oxygénant.

3. Tissu selon la revendication 1 ou 2, dans lequel ledit fil oxygénant est enroulé en spirale autour de ladite âme élastomère, de préférence le nombre d'enroulements dudit fil oxygénant autour de l'âme élastomère étant compris entre 400 et 800 enroulements par mètre.

4. Tissu selon l'une quelconque des revendications 1 à 3, dans lequel ledit fil oxygénant est un fil continu.

5. Tissu selon l'une quelconque des revendications précédentes, dans lequel ledit fil élastique et/ou ledit fil non-élastique comprend un fil doublé constitué de filaments de polymère oxygénant.

6. Tissu selon l'une quelconque des revendications précédentes, comprenant en outre un fil antibactérien ayant de préférence un nombre de fils de coton Anglais compris entre 84,4 et 492,1 dtex (12 et 70 Ne), de préférence entre 118,1 et 492,1 dtex (12 et 50 Ne), de manière encore plus préférée entre 196,8 et 295,3 dtex (20 et 30 Ne).

7. Tissu selon la revendication 6, dans lequel ledit fil antibactérien comprend une fibre comprenant au moins un dérivé de chitine, et dans lequel ledit dérivé de chitine est présent dans ledit fil antibactérien selon un pourcentage en poids égal ou supérieur à 20 %.

8. Tissu selon la revendication 6 ou 7, dans lequel ledit fil dudit polymère oxygénant est présent selon une quantité comprise entre 50 % et 99 % en poids/poids de l'ensemble du tissu, ledit fil élastomère à âme filée est présent selon une quantité comprise entre 1 % et 5 % en poids/poids et ledit fil antibactérien est présent selon une quantité comprise entre 0 % et 40 % en poids/poids.

9. Tissu selon l'une des revendications précédentes, ayant un poids compris dans la plage de 90 g/m² à 270 g/m², de préférence de 110 g/m² à 250 g/m².

10. Tissu selon la revendication 1, **caractérisé en ce que** ledit fil non-élastomère est un fil continu de polymère oxygénant, dans lequel les fibres de filament dudit polymère sont dans un état non entrelacé ou non contraint.

11. Vêtement constitué d'un tissu, ou comprenant celui-ci, selon l'une quelconque des revendications 1 à 10.

12. Fil élastique comprenant une âme élastomère et un fil non-élastomère, ledit fil non-élastomère étant disposé autour de ladite âme élastomère pour le recouvrir au moins partiellement, **caractérisé en ce que** ledit fil non-élastomère est un fil obtenu à partir de fibres de polymère oxygénant, adapté pour augmenter le taux d'oxygène dans le sang d'une personne portant ledit fil, ladite âme élastomère ayant une densité massique linéaire comprise dans la plage de 22,2 à 80,9 dtex (20 à 80 dens) et ledit fil non-élastomère ayant une densité massique linéaire comprise dans la plage de 11,1 à 155,6 dtex (10 et 140 dens).

13. Fil selon la revendication 12, dans lequel ledit fil non-élastomère recouvrant au moins partiellement l'âme élastomère est réalisé sous la forme d'un filament continu et est enroulé en spirale autour de ladite âme, de préférence avec un nombre d'enroulements par mètre dudit polymère oxygénant autour de l'âme élastomère compris dans la plage de 400 à 800 vrilles par mètre.

14. Fil selon la revendication 12, dans lequel le fil non-élastomère est un fil comprenant des fibres de polymère oxygénant et des fibres ayant des propriétés antibactériennes.

15. Fil selon la revendication 14, dans lequel la quantité desdites fibres de polymère oxygénant dans ledit fil est comprise dans une plage allant de 50 % à 80 % en poids/poids, et la quantité desdites fibres ayant des propriétés antibactériennes dans ledit fil est comprise dans une plage allant de 20 % à 50 % en poids/poids.

16. Fil selon l'une quelconque des revendications 12 à 15, qui est un fil à âme filée, dans lequel lesdites fibres de polymère oxygénant sont des fibres discontinues.

17. Vêtement comprenant un fil selon l'une quelconque des revendications 12 à 16.
